# FASCICULE DE BREVET EUROPEEN

(11) **EP 1 762 223 B1**
(45) Date de publication et mention de la délivrance du brevet: **13.04.2016**
(21) Numéro de dépôt: 06118506.2
(22) Date de dépôt: 07.08.2006
(51) Int. Cl.: A61K 8/81, A61K 8/87, A61K 8/49, A61Q 5/10

(54) **Composition de coloration des fibres kératiniques comprenant au moins une base d'oxydation et un ester de sorbitan polyoxyéthylène particulier**
Zusammensetzung zum Färben von Keratinfasern enthaltend mindestens ein Oxidationsmittel und einen besonderen Polyoxyethylensorbitan-Ester
Composition for dyeing keratin fibres comprising at least one oxidation base and a particular polyoxyethylenated sorbitan ester

(30) Priorité: 11.08.2005 FR 0552492
(43) Date de publication de la demande: 14.03.2007
(73) Titulaire: L'Oréal, 75008 Paris (FR)
(72) Inventeur: Audousset, Marie-Pascale, 92600 Asnières (FR); Rondeau, Christine, 78500 Sartrouville (FR)
(74) Mandataire: Fevrier, Murielle Françoise E.

(56) Documents cités:
- WO-A-2004/093835
- DE-A1- 19 923 438
- ANONYMOUS: "HYDROPHOBIC OXIDATIVE HAIR DYE 8781-58" HAIRCAREFORMULATION.COM, [Online] 11 février 2002 (2002-02-11), XP002380394 Extrait de l'Internet: URL:http://www.haircareformulation.com/Doc /EN/FORMULATION/878158.pdf> [extrait le 2006-05-10]
- PATENT ABSTRACTS OF JAPAN vol. 015, no. 388 (C-0872), 2 octobre 1991 (1991-10-02) & JP 03 157320 A (YAMAHATSU SANGYO KK), 5 juillet 1991 (1991-07-05)

## Description

La présente invention a pour objet une composition de coloration des fibres kératiniques qui comprend au moins une base d'oxydation et un ester de sorbitan polyoxyéthyléné particulier.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés. Les nuances obtenues avec ces bases d'oxydation peuvent être modifiées par l'addition de coupleurs ou modificateurs de coloration, ces derniers étant choisis notamment parmi les méta-diaminobenzènes aromatiques, les méta-aminophénols, les méta-diphénols et certains composés hétérocycliques tels que des composés indoliques et pyridiniques.

La coloration d'oxydation est généralement mise en oeuvre en présence d'agent alcalin qui favorise la coloration des fibres kératiniques.

La variété des molécules mises en jeu au niveau des bases d'oxydation et des coupleurs, permet l'obtention d'une riche palette de couleurs. La coloration dite "permanente" obtenue grâce à ces colorants d'oxydation, permet d'obtenir des nuances dans l'intensité souhaitée et présente une bonne tenue face aux agents extérieurs tels que la lumière, les intempéries, le lavage, les ondulations permanentes, la transpiration et les frottements.

Cependant, la coloration d'oxydation étant mise en oeuvre en présence d'un agent oxydant et d'agent alcalin, elle entraîne parfois une sensation d'inconfort se traduisant par des picotements et/ou des échauffements locaux du cuir chevelu.

Il est déjà connu de protéger les fibres kératiniques devant subir ou ayant subies une coloration à partir de précurseur de coloration notamment par l'utilisation de polymères particuliers. Cependant, cette protection n'est pas complètement satisfaisante, en particulier, elle peut entraîner une teinture moins puissante due à la présence de ces polymères.

Par ailleurs, il est déjà connu d'utiliser des esters de sorbitan polyoxyéthyléné dans des produits de coloration des fibres kératiniques. En particulier, le document DE 19923438 décrit l'utilisation d'esters de sorbitan polyoxyéthyléné pour réduire le tachâge du cuir chevelu au cours de la teinture.

Le but de la présente invention est de fournir une nouvelle composition de coloration des fibres kératiniques telles que les cheveux qui permet de limiter l'inconfort qui peut être ressenti par l'utilisatrice lors d'une coloration.

Ce but est atteint par la présente invention qui a pour objet une composition de coloration des fibres kératiniques tels que les cheveux qui comprend dans un milieu approprié au moins une base d'oxydation, au moins un coupleur et au moins un ester de sorbitan polyoxyéthyléné dont le nombre de moles d'oxyde d'éthylène varie de 2 à 5, bornes incluses.

Une telle composition permet notamment de conserver une coloration puissante tout en limitant l'inconfort pouvant être ressenti au niveau du cuir chevelu au moment de l'application de la composition de coloration ou après cette application.

Les bases d'oxydation utiles dans la composition de la présente invention des bases d'oxydations classiquement utilisées pour la coloration par oxydation.

A titre d'exemple, les bases d'oxydation peuvent être choisies parmi les para-phénylènediamines, les bis-phénylalkylènediamines, les para-aminophénols, les bis-para-aminophénols, les ortho-aminophénols, les bases hétérocycliques et leurs sels d'addition.

Parmi les para-phénylènediamines, on peut citer la para-phénylènediamine, la para-toluènediamine, la 2 chloro para-phénylènediamine, la 2,3-diméthyl para-phénylènediamine, la 2,6 diméthyl para-phénylènediamine, la 2,6-diéthyl para-phénylènediamine, la 2,5 diméthyl para-phénylènediamine, la N,N-diméthyl para-phénylènediamine, la N,N-diéthyl para-phénylènediamine, la N,N-dipropyl para-phénylènediamine, la 4 amino N,N diéthyl 3-méthyl aniline, la N,N-bis-(β-hydroxyéthyl) para-phénylènediamine, la 4-N,N-bis-(β-hydroxyéthyl)amino 2-méthyl aniline, la 4 N,N bis (β hydroxyéthyl)amino 2-chloro aniline, la 2 β hydroxyéthyl para-phénylènediamine, la 2 fluoro para-phénylènediamine, la 2 isopropyl para-phénylènediamine, la N (β hydroxypropyl) para-phénylènediamine, la 2 hydroxyméthyl para-phénylènediamine, la N,N diméthyl 3-méthyl para-phénylènediamine, la N,N (éthyl, β-hydroxyéthyl) para-phénylènediamine, la N-(β,γ-dihydroxypropyl) para-phénylènediamine, la N (4' aminophényl) para-phénylènediamine, la N phényl para-phénylènediamine, la 2 β hydroxyéthyloxy para-phénylènediamine, la 2-β-acétylaminoéthyloxy para-phénylènediamine, la N (β méthoxyéthyl) para-phénylène-diamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2 isopropyl para-phénylènediamine, la 2-β-hydroxyéthyl para-phénylènediamine, la 2 β hydroxyéthyloxy para-phénylène-diamine, la 2,6-diméthyl para-phénylènediamine, la 2,6-diéthyl para-phénylènediamine, la 2,3 diméthyl para-phénylènediamine, la N,N bis-(β-hydroxyéthyl) para-phénylènediamine, la 2 chloro para-phénylènediamine, la 2 β acétylaminoéthyloxy para-phénylènediamine, et leurs sels d'addition avec un acide sont particulièrement préférées.

Parmi les bis-phénylalkylènediamines, on peut-citer à titre d'exemple, le N,N'-bis-(β-hydroxyéthyl) N,N'-bis-(4'-aminophényl) 1,3 diamino propanol, la N,N'-bis-(β-hydroxyéthyl) N,N'-bis-(4'-aminophényl) éthylènediamine, la N,N' bis-(4-aminophényl) tétraméthylènediamine, la N,N' bis (β hydroxyéthyl) N,N'-bis-(4-aminophényl) tétraméthylènediamine, la N,N' bis-(4-méthyl-aminophényl) tétraméthylènediamine, la N,N'-bis-(éthyl) N,N' bis (4'-amino, 3' méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4 amino 3 fluoro phénol, le 4 amino 3-hydroxyméthyl phénol, le 4-amino 2-méthyl phénol, le 4-amino 2 hydroxyméthyl phénol, le 4-amino 2-méthoxyméthyl phénol, le 4 amino 2 aminométhyl phénol, le 4-amino 2-(β-hydroxyéthyl aminométhyl) phénol, le 4 amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6 méthyl phénol, le 5-acétamido 2 amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques tels que les dérivés 4,5-diamino pyrazoles.

Parmi les dérivés pyridiniques, on peut citer les composés décrits par exemple dans les brevets GB 1 026 978 et GB 1 153 196, comme la 2,5 diamino pyridine, la 2-(4-méthoxyphényl)amino 3 amino pyridine, la 2,3 diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5 a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-{2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo(1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88 169571 ; JP 05-63124 ; EP 0770375 ou demande de brevet WO 96/15765 comme la 2,4,5,6-tétra-aminopyrimidine, la 4 hydroxy 2,5,6-triaminopyrimidine, la 2 hydroxy 4,5,6-triaminopyrimidine, la 2,4 dihydroxy 5,6-diaminopyrimidine, la 2,5,6 triaminopyrimidine, et les dérivés pyrazolo-pyrimidiniques tels ceux mentionnés dans la demande de brevet FR A 2750048 et parmi lesquels on peut citer la pyrazolo-[1,5-a]-pyrimidine-3,7-diamine ; la 2,5-diméthyl pyrazolo [1,5 a]-pyrimidine-3,7-diamine ; la pyrazolo-[1,5-a]-pyrimidine-3,5-diamine ; la 2,7 diméthyl pyrazolo-[1,5-a]-pyrimidine-3,5-diamine ; le 3-amino pyrazolo-[1,5 a]-pyrimidin-7-ol ; le 3-amino pyrazolo-[1,5-a]-pyrimidin-5-ol ; le 2(3 amino pyrazolo-[1,5-a]-pyrimidin-7-ylamino)-éthanol, le 2 (7-amino pyrazolo-[1,5-a]-pyrimidin-3-ylamino)-éthanol, le 2-[(3-amino-pyrazolo[1,5-a]pyrimidin-7-yl)-(2 hydroxy-éthyl)-amino]-éthanol, le 2-[(7-amino-pyrazolo[1,5-a]pyrimidin-3-yl)-(2 hydroxy-éthyl)-amino]-éthanol, la 5,6-diméthyl pyrazolo-[1,5-a]-pyrimidine-3,7 diamine, la 2,6 diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5 diamino 1-méthyl pyrazole, le 4,5-diamino 1-(β-hydroxyéthyl) pyrazole, le 3,4 diamino pyrazole, le 4,5-diamino 1 (4' chlorobenzyl) pyrazole, le 4,5-diamino 1,3-diméthyl pyrazole, le 4,5 diamino 3-méthyl 1-phényl pyrazole, le 4,5-diamino 1-méthyl 3-phényl pyrazole, le 4-amino 1,3-diméthyl 5-hydrazino pyrazole, le 1 benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1 (β hydroxyéthyl) 3-méthyl pyrazole, le 4,5 diamino 1 éthyl 3-méthyl pyrazole, le 4,5-diamino 1-éthyl 3 (4' méthoxyphényl) pyrazole, le 4,5-diamino 1-éthyl 3 hydroxyméthyl pyrazole, le 4,5-diamino 3-hydroxyméthyl 1 méthyl pyrazole, le 4,5-diamino 3 hydroxyméthyl 1-isopropyl pyrazole, le 4,5 diamino 3-méthyl 1 isopropyl pyrazole, le 4-amino 5-(2'-aminoéthyl)amino 1,3 diméthyl pyrazole, le 3,4,5 triamino pyrazole, le 1-méthyl 3,4,5-triamino pyrazole, le 3,5-diamino 1 méthyl 4-méthylamino pyrazole, le 3,5-diamino 4 (β hydroxyéthyl)amino 1 méthyl pyrazole, et leurs sels d'addition avec un acide.

De préférence, les compositions de l'invention contiennent au moins une para-phénylènediamine et/ou un para-aminophénol.

La ou les bases d'oxydation présentes dans la composition de coloration de l'invention sont en général présentes chacune en quantité comprise entre 0,001 à 10 % en poids environ du poids total de la composition tinctoriale, de préférence entre 0,005 et 6 %.

La composition de l'invention contient un coupleur. De tels coupleurs sont des coupleurs classiquement utilisés dans le cadre de la coloration d'oxydation tels que les coupleurs métaphénylènediamines, les coupleurs méta-aminophénols, les coupleurs métadiphénols, les coupleurs naphtaléniques, les coupleurs hétérocycliques et leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de coloration de la présente invention, le-ou les coupleurs, lorsqu'ils sont présents, sont chacun généralement présents en quantité comprise entre 0,001 et 10 % en poids environ du poids total de la composition tinctoriale, de préférence entre 0,005 et 6 %.

A titre d'exemple d'ester de sorbitan polyoxyéthyléné dont le nombre de moles d'oxyde d'éthylène varie de 2 à 5, bornes incluses utile dans la composition de l'invention, on peut citer le mono-laurate de sorbitan oxyéthyléné à 4OE ou polysorbate 21, le mono-stéarate de sorbitan oxyéthylénéà 4OE ou polysorbate 61, le mono-oléate de sorbitan oxyéthyléné à 5OE ou polysorbate 81. Ces esters de sorbitan sont par exemple commercialisés par la société Uniquema sous la dénomination Tween 21, Tween 61 ou Tween 81.

Selon un mode de réalisation particulier, le nombre de moles d'oxyde d'éthylène est de préférence inférieur à 6 moles d'oxyde d'éthylène, et de préférence variant de 2 à 5 moles d'oxyde d'éthylène, bornes incluses.

Selon la présente invention, l'ester de sorbitan peut être présent dans la composition en quantité très variable en fonction par exemple du type de base d'oxydation ou de la nature des fibres kératiniques à teindre. Selon un mode de réalisation particulier, la composition de l'invention peut contenir une quantité d'ester de sorbitan variant de 0,01 à 20 % en poids par rapport au poids de la composition, plus particulièrement une quantité variant de -0,1 à 10% et de préférence une quantité variant de 1 à 8 %.

La composition de coloration peut de plus comprendre des colorants directs. A titre de colorant direct, on peut citer les colorants directs nitrés benzéniques neutres, acides ou cationiques, les colorants directs azoiques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Le milieu approprié pour la coloration est avantageusement un milieu cosmétique constitué par de l'eau ou par un mélange d'eau et d'au moins un solvant organique tel que, par exemple, les alcools inférieurs en C1-C4, ramifiés ou non, tels que l'éthanol et l'isopropanol ; les polyols et éthers de polyols comme le 2-butoxyéthanol, le propylèneglycol, le monométhyléther de propylèneglycol, le onoéthyléther et le monométhyléther du diéthylèneglycol, le glycérol ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en- poids environ.

La composition de coloration utile dans le procédé de l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes et en particulière des polymères fixants non ioniques, cationiques, anioniques, amphotères, des agents conservateurs, des agents opacifiants.

Selon un mode de réalisation particulier, la composition de l'invention contient au moins un agent tensio-actif, de préférence non ionique et/ou un polymère épaississant.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels adjuvants de manière telle que les propriétés avantageuses attachées intrinsèquement à la composition conforme à l'invention ne soient pas, ou substantiellement pas, altérées par la ou les adjonctions envisagées.

Le pH de la composition de coloration est généralement compris entre 2 et 12 environ, de préférence compris entre 6 et 12.

Il peut être ajusté à la valeur désirée au moyen d'agents acidifiants ou alcalinisants habituellement utilisés en coloration des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 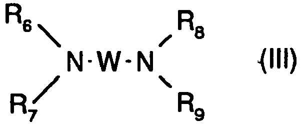 dans laquelle W est un reste propylène éventuellement substitué par un groupement hydroxyle ou un radical alkyle en C₁-C₄ ; R₆, R₇, R₈ et R₉, identiques ou différents, représentent un atome d'hydrogène, un radical alkyle en C₁-C₄ ou hydroxyalkyle en C₁-C₄.

La composition de coloration est généralement mise en oeuvre à partir d'une composition contenant un agent alcalin, cet agent alcalin étant en général présent en quantité supérieure à 5 % en poids par rapport au poids de la composition de coloration. Il peut être présent en des quantités supérieures à 10 %, en particulier supérieures à 15 %.

Selon un mode de réalisation particulier de l'invention, la composition de coloration comprend ou est destinée à être mise en oeuvre avec au moins un agent alcalinisant, notamment de l'ammoniaque et/ou une alcanolamine tel que l'éthanolamine et/ou un silicate tel que le silicate de sodium.

La composition de coloration selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La composition de coloration peut de plus comprendre un agent oxydant. Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

L'agent oxydant peut être ajouté à la composition de coloration juste au moment de l'emploi ou il peut être mis en oeuvre à partir d'une composition oxydante le contenant, cette composition étant appliquée simultanément ou séquentiellement à la composition de l'invention. La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 6 et 12. Il peut être ajusté à la valeur désirée au moyen d'agents acidifiants ou alcalinisants habituellement utilisés en teinture des fibres kératiniques et tels que définis précédemment.

La composition de coloration qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

La coloration des fibres kératiniques est obtenue de façon classique par application de la composition de coloration pendant un temps suffisant pour obtenir la coloration désirée. Le temps de pose est généralement compris entre 1 à 60 minutes environ, de préférence 5 à 60 minutes environ. Cette étape de coloration est généralement suivie par une étape de rinçage.

L'étape de coloration peuvent être mis en oeuvre à température ambiante ou à des températures plus élevées par exemple en utilisant un sèche cheveux, un casque de séchage, un fer à lisser, etc.

Les exemples suivants sont destinés à illustrer l'invention. Celle-ci n'est cependant pas limitée à ces modes de réalisation.

### EXEMPLES

On a préparé la composition de colorations suivantes (en grammes)

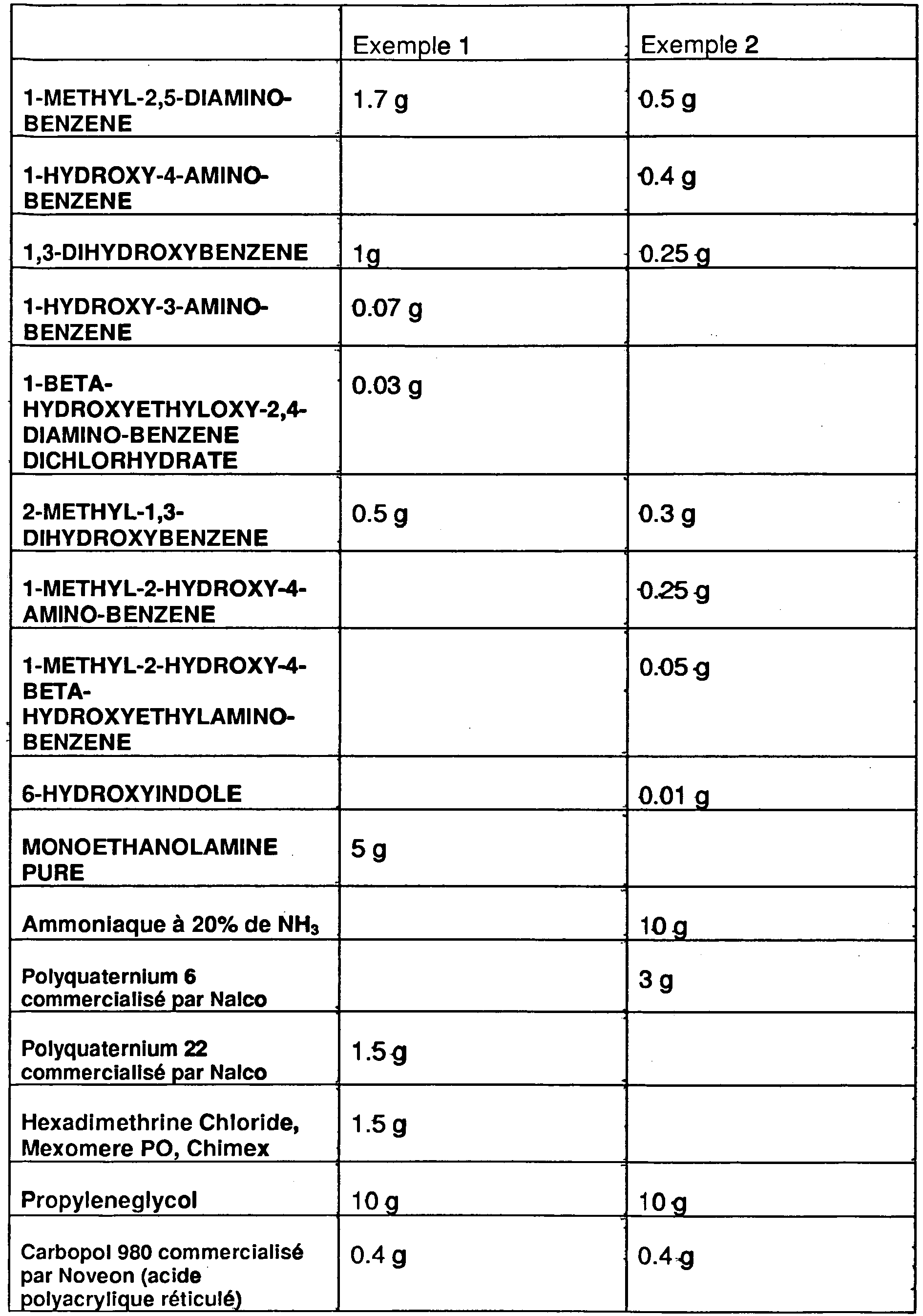

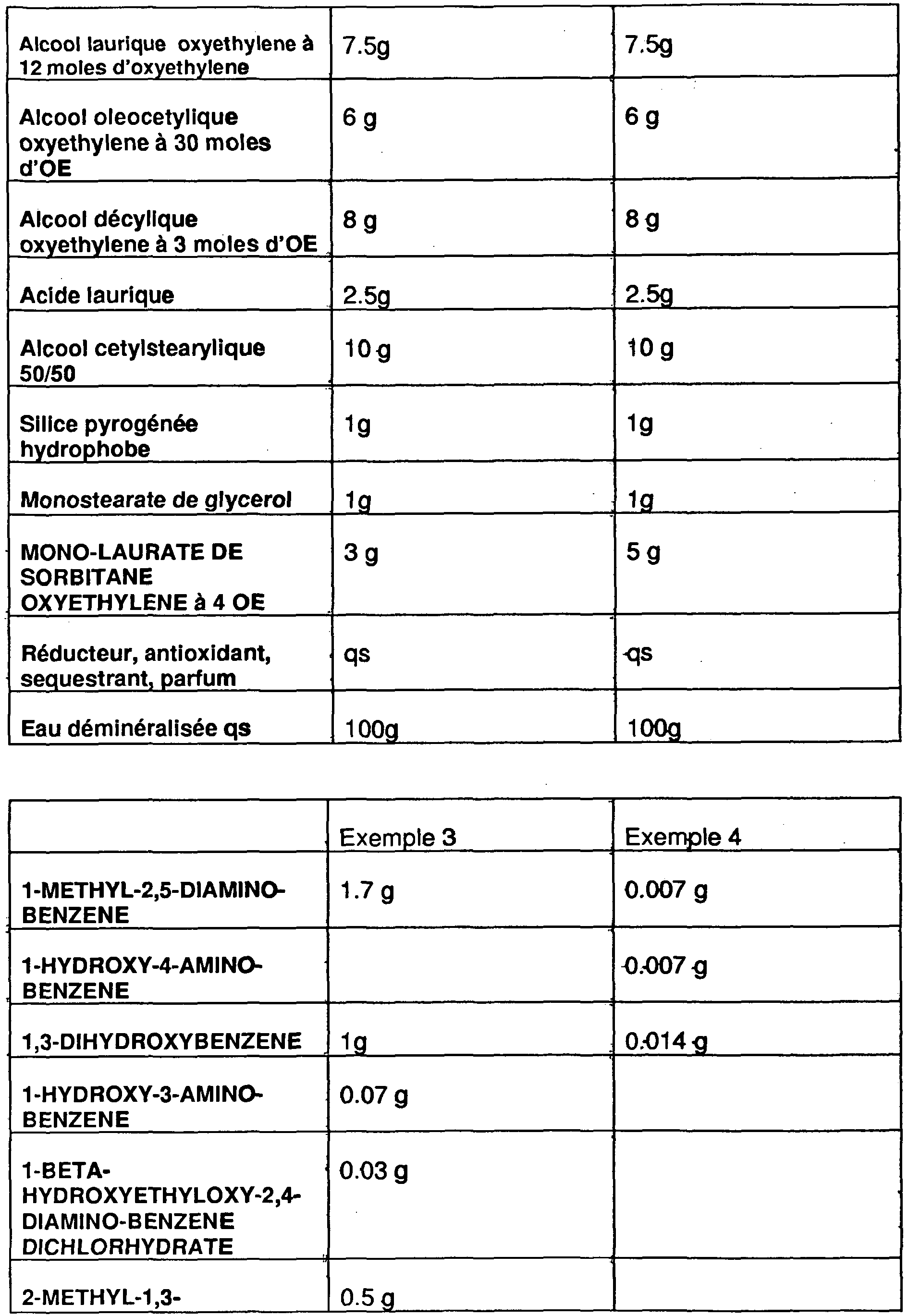

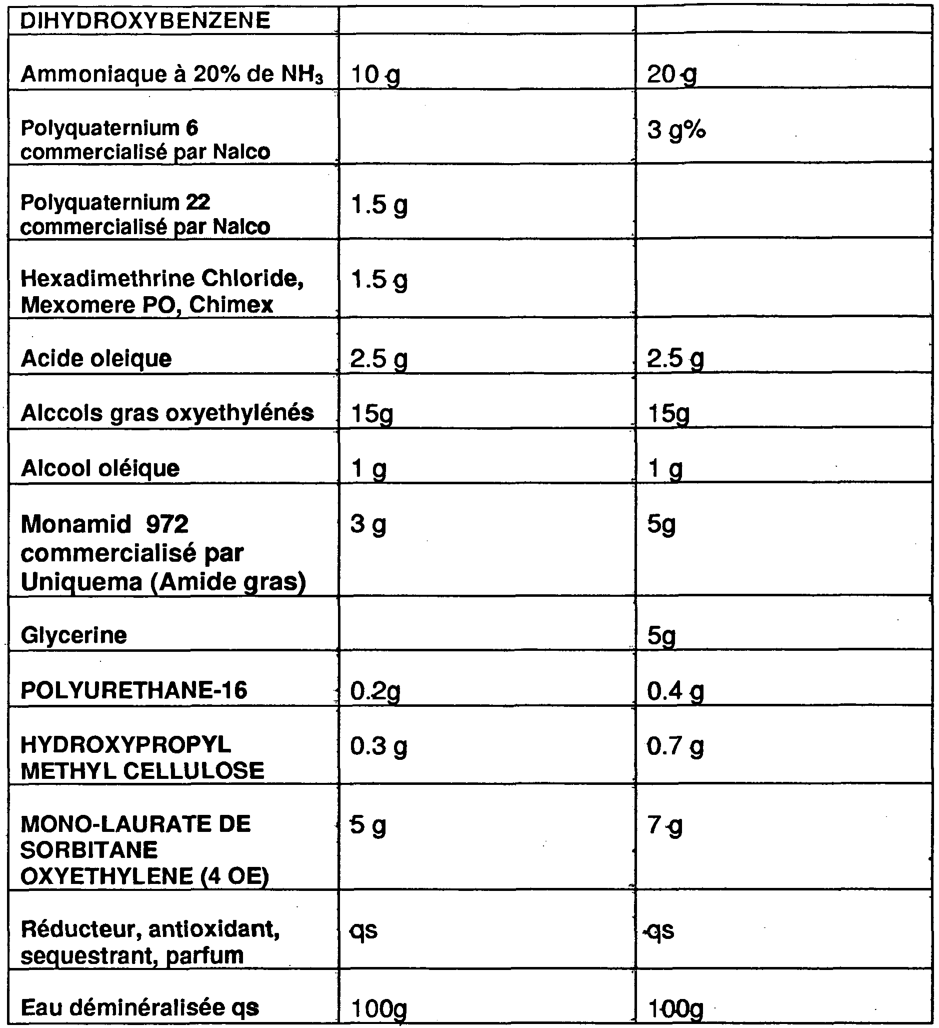

La composition 1 est mélangée extemporanément avec 1 fois et demi son volume d'eau oxygénée (pH voisin de 3) à 9 volumes.

La composition 2 est mélangée extemporanément avec 1 fois et demi son volume d'eau oxygénée à 20 volumes.

Les compositions 3 et 4 sont mélangées extemporanément avec 1 fois-et demi son volume d'eau oxygénée (pH voisin de 3) à 20 volumes pour la composition 3 et avec 2 fois leur volume d'eau oxygénée (pH voisin de 3) à 40 volumes pour la composition 4.

Chaque mélange ainsi obtenu est appliqué sur des cheveux gris à 90% de blancs, à raison de 30 g pour 3 g de cheveux. Le temps de pose à température ambiante est de 20 min pour les compositions 1 et 2 et de 30 min pour les compositions 3 et 4.

Les cheveux sont ensuite rincés, lavés avec un shampooing standard et séchés. La coloration capillaire est évaluée de manière visuelle.

| | **Hauteur de ton** | **Reflet** |
|---|---|---|
| composition **1** | Châtain | naturel |
| composition **2** | Blond foncé | Cuivré acajou |
| composition **3** | Châtain | naturel |
| composition **4** | Blond très très clair | naturel |

Les fibres ainsi obtenues présentent une coloration satisfaisante dans de bonnes conditions de confort pour le modèle.

## Revendications

1. Composition de coloration des fibres kératiniques qui comprend dans un milieu approprié au moins une base d'oxydation au moins un coupleur et au moins un ester de sorbitan polyoxyéthyléné dont le nombre de moles d'oxyde d'éthylène varie de 2 à 5, bornes incluses.

2. Composition selon la revendication 1 dans laquelle l'ester de sorbitan polyoxyéthyléné est choisi parmi le mono-laurate de sorbitan oxyéthyléné à 4OE, le mono-stéarate de sorbitan oxyéthyléné à 4OE, le mono-oléate de sorbitan oxyéthyléné à 5OE.

3. Composition selon l'une quelconque des revendications précédentes dans laquelle la quantité d'ester de sorbitan varie de 0,01 à 20 % en poids par rapport au poids de la composition, de préférence de 0,1 à 10 %.

4. Composition selon l'une quelconque des revendications 1 à 3 dans laquelle la quantité d'ester de sorbitan varie entre 1 et 8 %.

5. Composition selon l'une quelconque des revendications précédentes dans laquelle la ou les bases d'oxydation sont choisies parmi les para-phénylènediamines, les bis-phénylalkylènediamines, les para-aminophénols, les bis-para-aminophénols, les ortho-aminophénols, les bases hétérocycliques et leurs sels d'addition.

6. Composition selon la revendication 5 dans laquelle la ou les bases d'oxydation présentes dans la composition de coloration sont présentes chacune en quantité comprise entre 0,001 à 10 % en poids environ du poids total de la composition, de préférence entre 0,005 et 6 %

7. Composition selon l'une quelconque des revendications précédentes dans laquelle la composition de coloration comprend au moins un coupleur choisi parmi les coupleurs méta-phénylènediamines, les coupleurs méta-aminophénols, les coupleurs méta-diphénols, les coupleurs naphtaléniques, les coupleurs hétérocycliques et leurs sels d'addition.

8. Composition selon la revendication 7 dans laquelle le ou les coupleurs sont chacun présents en quantité comprise entre 0,001 et 10 % en poids environ du poids total de la composition tinctoriale, de préférence entre 0,005 et 6 %

9. Composition selon l'une quelconque des revendications précédentes dans laquelle la composition de coloration comprend de plus un colorant direct

10. Composition selon l'une quelconque de revendications précédentes dans laquelle la composition de coloration contient un agent alcalinisant.

11. Procédé de coloration des fibres kératiniques qui comprend l'application de la composition telle que définie à l'une quelconque des revendications 1 à 10 précédentes sur les fibres kératiniques pendant un temps suffisant pour obtenir la coloration désirée.

12. Utilisation d'un ester de sorbitan polyoxyéthyléné dont le nombre de moles d'oxyde d'éthylène varie de 2 à 5, bornes incluses dans une composition contenant au moins une base d'oxydation et au moins un coupleur pour supprimer ou diminuer les sensations d'inconfort liés à l'application sur le cuir chevelu de la composition de coloration en présence d'un agent oxydant.

## Patentansprüche

1. Zusammensetzung zum Färben von Keratinfasern, die in einem geeigneten Medium mindestens eine Oxidationsbase, mindestens einen Kuppler und mindestens einen polyoxyethylenierten Sorbitanester, in dem die Zahl der Mole Ethylenoxid im Bereich von 2 bis 5 einschließlich der Grenzen liegt.

2. Zusammensetzung nach Anspruch 1, wobei der polyoxyethylenierte Sorbitanester aus oxyethyleniertem Sorbitanmonolaurat mit 4 EO, oxyethyleniertem Sorbitanmonostearat mit 4 EO und oxyethyleniertem Sorbitanmonooleat mit 5 EO ausgewählt ist.

3. Zusammensetzung nach einem der vorhergehenden Ansprüche, wobei die Menge an Sorbitanester im Bereich von 0,01 bis 20 Gew.-%, vorzugsweise 0,1 bis 10 Gew.-%, bezogen auf das Gewicht der Zusammensetzung, liegt.

4. Zusammensetzung nach einem der Ansprüche 1 bis 3, wobei die Menge an Sorbitanester im Bereich von 1 bis 8% liegt.

5. Zusammensetzung nach einem der vorhergehenden Ansprüche, wobei die Oxidationsbase bzw. die Oxidationsbasen aus para-Phenylendiaminen, Bisphenylalkylendiaminen, para-Aminophenolen, Bis-para-aminophenolen, o-Aminophenolen, heterocyclischen Basen und Additionssalzen davon ausgewählt sind.

6. Zusammensetzung nach Anspruch 5, wobei die Oxidationsbase bzw. die Oxidationsbasen in der Färbezusammensetzung jeweils in einer Menge zwischen ungefähr 0,001 bis 10 Gew.-%, vorzugsweise zwischen 0,005 und 6 Gew.-%, des Gesamtgewichts der Zusammensetzung, vorliegen.

7. Zusammensetzung nach einem der vorhergehenden Ansprüche, wobei die Färbezusammensetzung mindestens einen Kuppler, der aus meta-Phenylendiamin-Kupplern, meta-Aminophenol-Kupplern, meta-Diphenol-Kupplern, Naphthalin-Kupplern, heterocyclischen Kupplern und Additionssalzen davon ausgewählt ist, umfasst.

8. Zusammensetzung nach Anspruch 7, wobei der Kuppler bzw. die Kuppler jeweils in einer Menge zwischen ungefähr 0,001 bis 10 Gew.-%, vorzugsweise zwischen 0,005 und 6 Gew.-%, des Gesamtgewichts der Färbezusammensetzung, vorliegen.

9. Zusammensetzung nach einem der vorhergehenden Ansprüche, wobei die Färbezusammensetzung außerdem einen Direktfarbstoff umfasst.

10. Zusammensetzung nach einem der vorhergehenden Ansprüche, wobei die Färbezusammensetzung ein Alkalinisierungsmittel enthält.

11. Verfahren zum Färben von Keratinfasern, bei dem man die Zusammensetzung gemäß einem der vorhergehenden Ansprüche 1 bis 10 über einen zum Erhalt der gewünschten Färbung ausreichenden Zeitraum auf die Keratinfasern aufbringt.

12. Verwendung eines polyoxyethylenierten Sorbitanesters, in dem die Zahl der Mole Ethylenoxid im Bereich von 2 bis 5 einschließlich der Grenzen liegt, in einer Zusammensetzung, die mindestens eine Oxidationsbase und mindestens einen Kuppler enthält, zur Unterdrückung oder Verringerung der mit dem Aufbringen der Färbezusammensetzung in Gegenwart eines Oxidationsmittels auf die Kopfhaut verbundenen Unbehaglichkeitsempfindungen.

## Claims

1. Composition for dyeing keratin fibres, which comprises, in a suitable medium, at least one oxidation base, at least one coupler and at least one polyoxyethylenated sorbitan ester with a number of moles of ethylene oxide ranging from 2 to 5, limits inclusive.

2. Composition according to Claim 1, in which the polyoxyethylenated sorbitan ester is chosen from sorbitan monolaurate oxyethylenated with 4 EO, sorbitan monostearate oxyethylenated with 4 EO and sorbitan monooleate oxyethylenated with 5 EO.

3. Composition according to either of the preceding claims, in which the amount of sorbitan ester ranges from 0.01% to 20% and preferably from 0.1% to 10% by weight relative to the weight of the composition.

4. Composition according to any one of Claims 1 to 3, in which the amount of sorbitan ester ranges between 1% and 8%.

5. Composition according to any one of the preceding claims, in which the oxidation base(s) is (are) chosen from para-phenylenediamines, bis(phenyl)alkylenediamines, para-aminophenols, bis-para-aminophenols, ortho-aminophenols and heterocyclic bases, and the addition salts thereof.

6. Composition according to Claim 5, in which the oxidation base(s) present in the dye composition is (are) each present in an amount of between 0.001% and 10% and preferably between 0.005% and 6% by weight approximately relative to the total weight of the composition.

7. Composition according to any one of the preceding claims, in which the dye composition comprises at least one coupler chosen from meta-phenylenediamine couplers, meta-aminophenol couplers, meta-diphenol couplers, naphthalene-based couplers and heterocyclic couplers, and the addition salts thereof.

8. Composition according to Claim 7, in which the coupler(s) is (are) each present in an amount of between 0.001% and 10% and preferably between 0.005% and 6% by weight approximately relative to the total weight of the dye composition.

9. Composition according to any one of the preceding claims, in which the dye composition also comprises a direct dye.

10. Composition according to any one of the preceding claims, in which the dye composition contains a basifying agent.

11. Process for dyeing keratin fibres, which comprises the application of the composition as defined in any one of the preceding Claims 1 to 10 to the keratin fibres for a time that is sufficient to obtain the desired coloration.

12. Use of a polyoxyethylenated sorbitan ester with a number of moles of ethylene oxide ranging from 2 to 5, limits inclusive, in a composition containing at least one oxidation base and at least one coupler, to eliminate or reduce the sensations of discomfort associated with the application to the scalp of the dye composition in the presence of an oxidizing agent.
